(19) Europäisches Patentamt
European Patent Office
Office européen des brevets

(11) Publication number: **0 407 800 A2**

(12) # EUROPEAN PATENT APPLICATION

(21) Application number: **90112082.4**

(22) Date of filing: **26.06.90**

(51) Int. Cl.5: **G01N 33/52**, //C12Q1/00, C12Q1/54

(30) Priority: **08.07.89 DE 3922495**

(43) Date of publication of application: **16.01.91 Bulletin 91/03**

(84) Designated Contracting States: **DE FR GB IT**

(71) Applicant: **MILES INC.**
**1127 Myrtle Street**
**Elkhart,IN 46515(US)**

(72) Inventor: **Hildenbrand, Karlheinz, Dr.**
**Gatzenstrasse 147**
**D-4150 Krefeld(DE)**
Inventor: **Wehling, Klaus, Dr.**
**Am Rohm 121**
**D-5600 Wuppertal(DE)**
Inventor: **Pudleiner, Heinz, Dr.**
**Bethelstrasse 16**
**D-4150 Krefeld(DE)**
Inventor: **Engelhard, Helmut, Dr.**
**Moltkestrasse 9**
**D-4047 Dormagen(DE)**

(74) Representative: **Dänner, Klaus, Dr. et al**
**c/o Bayer AG Konzernverwaltung RP Patente Konzern**
**D-5090 Leverkusen 1 Bayerwerk(DE)**

(54) **Analysis method for determining substances from biological fluids.**

(57) A test strip for analyzing substances from biological fluids is disclosed in which the matrix comprises a porous membrane having an asymmetric pore structure which is designed for application of the biological fluid to the large pore side of the membrane and in which a determination of the biological fluid is made on the opposite side of the membrane containing a small pore size. The membrane contains anionic surfactant in an amount of 1 to about 4% by weight based on the polymer casting solution used to form the porous membrane.

EP 0 407 800 A2

# ANALYSIS METHOD FOR DETERMINING SUBSTANCES FROM BIOLOGICAL FLUIDS

## Field Of The Invention

The present invention relates to test strips or test devices for analyzing substances from biological fluids in which the matrix comprises a porous membrane having asymmetric pore structure and designed for the application of the biological fluid to the large pore side of the membrane and making a determination on the opposite fine pore side of the membrane. The membrane contains anionic surfactant equivalent to surfactant addition of about 1 to about 4 percent by weight based on polymer casting solution.

## Background Of The Invention

Test strips which contain reagents in a matrix of paper or plastic material and in which the sample is applied directly to this matrix have become extremely important for quick and simple analysis of individual samples. Measurement results which are the same as or better than wet chemical methods can be obtained.

However, the test strips commercially available to date for determining blood constituents frequently possess certain disadvantages. The erythrocytes contained in blood interfere with most methods. The user must therefore typically remove serum or plasma from whole blood by centrifuging before making an analysis. This, however, presents problems, particularly in the case of small sample amounts. With more recent testing agents the reagent layer itself (European Patent 0 064 710, DOS German Published Specification 34 07 395) or a covering membrane is semipermeable and retains the erythrocytes. Accordingly, the test systems can be directly loaded with whole blood. However, in these test systems hemoglobin has to be removed by wiping or washing off before reflection-photometry or visual analysis. This method cannot be used for analysis of large molecules, e.g., certain enzymes, because these are also retained by the semipermeable layer. In addition, the wiping-off process constitutes a potential source of dangerous infections because blood samples may occasionally be contaminated with hepatitis viruses or other pathogens.

In view of the requirement for high reproducibility of the diagnosis, test strips systems which have a very simple structure and are uncomplicated to produce, with a few production steps, are preferred.

It is occasionally also desirable to have a delay phase between application of the sample and start of the reaction so that the temperature of the sample liquid can be stabilized before any subsequent reactions are carried out.

Finally, it is desirable to be able to use the same matrix for analysis of blood and urine. In fact, as described in detail in EP-A 64 710 and DE 38 90 523.8, the previously known microporous blood matrices cannot be used directly, without further aids, for a dip-and-read test in urinalysis.

There is as yet no test strip system which fulfills all these requirements simultaneously.

There has been no lack of attempts aimed at developing test strips for blood analyses in which separation of the erythrocytes or hemoglobin is simultaneously accomplished in the system.

DE-AS (German Published Specification) 22 22 951 or U.S. Patent No. 4,144,306 describes multilayer test devices for blood in which one or more of these layers act as filters which retain the corpuscular constituents of blood. Membranes having pores of 1-3 $\mu$m are used for separation of the erythrocytes. These pores easily block and impede passage of plasma with the result that the plasma enters the reaction layer slowly and nonuniformly.

Progress was achieved by using special glass fiber filters as described in DE-OS (German Published Specification) 30 29 579.5. On application of blood the particulate constituents of blood are retained while the plasma is transported to the reagent layer in which the detection reaction takes place. However, the dead volume of this erythrocyte retention zone is relatively high. The ratio of separated plasma to dosed blood therefore becomes even worse. Thus, it can occasionally be the case that the separation system cannot cope with the quantity of erythrocytes to be separated off, so that blood pigment reaches the reaction zone and causes interference there.

Special retention systems are described in European Patent 0 133 895 for further improvement of this glass fiber system. These are, for example, papers impregnated with polar compounds. e.g., special dyes. These substrates effect strong coagulation of blood, so that the corpuscular constituents are separated more effectively from the serum.

As described in European Patent 0 133 895, there is no universal retention substrate which is equally

suitable for each test. For example, some substances cause hemolysis or undergo undesirable secondary reactions with enzymes. It is therefore necessary to determine the most favorable retention substrate for each test. In addition, some of the analytes to be determined can be entrapped when the blood coagulates. The structure of the systems described in European Patent 0 133 895 is fairly complicated. Thus, the system for detection of glucose consists, for example, of seven individual elements. The reaction is triggered by the polymer matrix, which is provided with the detection reagents, being pressed onto the transport wadding filled with plasma. Since an oxygen deficiency problem would arise in oxidation reactions in the compressed state specific reagent matrices have been developed for this system and are described in European Patent 0 113 896. These are film layers containing reagents on a multifilament fabric carrier layer or wadding. The reagent films are prepared, as described in this application, from aqueous polymer dispersions in the presence of fillers. Since a certain amount of oxygen is stored in the multifilament fabric layer an oxygen-consuming reaction can take place in the above system even in the compressed state.

A disadvantage of this system is that the oxygen content is limited and, as described in European Patent 0 113 896, is insufficient for the oxygen requirements essential for the reaction. The reaction layers on multifilament fabric layers described in this specification are used in combination with the above-mentioned erythrocyte retention substrates. Erythrocyte separation inherent to the system is evidently impossible with these reagent layers supported by wadding or fabric. As mentioned there, the reagent layers are prepared from polymer dispersions in the presence of fillers by the methods described in DE-AS (German Published Specification) 15 98 153, DE-OS (German Published Specification) 29 10 134 and DE-OS (German Published Specification) 31 18 381. The resulting pore structures have very small pore diameters, low porosities and no pore through-channels.

European Patent Applications 0 110 173 and 0 256 806 of Lifescan describe detection elements which have a considerably simpler and less complicated structure and which can be used directly for whole blood analysis. As described in the last-mentioned specification, the reagent matrix is hydrophilic polyamide membrane (nylon) impregnated with detection reagents.

A noteworthy feature of this system, which is already commercially available, is that the red blood pigments are not separated. On application of blood the entire reagent matrix becomes red. The glucose color reaction is evaluated with a device whose wavelength does not interfere with the red blood pigment but which detects the chromogenic reaction. However, these systems cannot be used for visual evaluations. As shown by analyses of this reagent matrix under the electron microscope, there are highly porous membrane layers located on either side of a polymer wadding. The pore structure is symmetrical and has diameters of 2 to 12 $\mu$m (microns). There can be no separation of erythrocytes. By contrast, it is described in the same application (EP-A 0 256 806) that membranes having mean pore diameters of 0.1 to 2.0 $\mu$m are preferably used for blood analyses. The above-mentioned difficulties apparently also occur with this system, i.e., that porous systems having pore diameters of less than 3 $\mu$m separate the erythrocytes but are blocked by high molecular compounds and, moreover, do not allow high molecular analytes to pass, whereas precisely the opposite is the case with membranes having much larger pores.

## Summary Of The Invention

It is an objection of the present invention, then, to develop a test strip system and an associated method of analysis whose construction and manipulation are as uncomplicated as the last-described system but which at the same time allows separation of red blood pigments (cells) without any additional aids (coagulants). In addition, it should be possible to precede the actual detection reaction, where necessary, by specific preliminary reactions, for example, to remove interfering components, it being possible for the detection and preliminary reactions to be separated in time, where necessary, by timing delays. Finally, it should furthermore be possible to use the detection elements for a dip-and-read test in urinalysis.

It has now been found, surprisingly, that membranes having an asymmetric pore structure into which the corresponding detection reagents are incorporated can be employed in an outstanding manner to achieve the aforementioned objects. The expression "asymmetric pore structure" is familiar to membrane experts and is described in detail in a large number of publications.

DOS 34 07 359 and U.S. Letters Patent No. 4,774,192 disclose a form of asymmetric membrane pore structure. However, in contrast to the claimed invention, sample is applied to the small pore side of the membrane. This is a sample receptive surface which the '192 patent described as "essentially impermeable to cells and particulate matter". Accordingly, in the case of blood and other biological samples the pores of the membrane become clogged very quickly rendering the membrane ineffective as a reagent matrix material for use with biological materials such as whole blood. Such detection systems are unsuitable for

the application purpose according to the invention, and are suitable only for wipe-off systems in which the blood has to be wiped off.

As described herein deficiencies of the prior art with respect to color gradation and color stability were overcome by the addition of certain anionic surfactants in particular amounts. These particular surfactants and the critical amounts of the surfactants are not disclosed by the '192 patent.

Two other publications which can be mentioned which refer to asymmetric pore structure and membranes are the following: H. Strathmann, "Trennung von molekularen Mischungen mit Hilfe synthetischer Membranen", (Separation of molecular mixture by means of synthetic membranes) Steinkopf-Verlag, Darmstadt 1979 and D. R. Lloyd, "Materials Science of Synthetic Membranes", ACS Symposium 269, Washington, D.C. 1985. The most important method of preparing such membranes, namely the precipitation coagulation method (also called phase inversion), is also mentioned. The integral asymmetric pore structure with a dense polymer structure, also called a polymer skin or active separating layer, at the membrane surface and larger pores and higher porosity in the underlying highly porous support layer are typical of such membranes.

Whereas the thickness of the active separating layer is about 0.2-2.9 µm, the underlying highly porous support layer is as a rule about 20 to 100 µm thick, depending on the wet application during production of the membrane. The structure of the highly porous support layer can possess a foam-like or finger-like structure or even a mixed structure of these two, depending on the polymer and the preparation method.

Subsequent modification or preparation with a further, e.g., even denser, active separating layer on the original active separating layer by the so-called composite method (J.E. Cadotte, ACS Symposium Ser. 153 [1981], 305-326) is also possible, but less preferred, for the preparation of the detection elements according to the invention.

The membranes used for preparation of the detection elements according to the invention can either be carrier-free or can adhere with the lower side of the membrane on a permeable carrier material customary for technical membranes, for example, polymer wadding or multifilament fabric. If suitable detection reagents are incorporated in such membrane systems and exposed to whole blood on the lower side of the membrane, the red blood pigments are separated in the membrane while a color reaction, without erythrocyte interference, can be observed on the opposite membrane side.

The pore size on the lower side of the membrane (application side) should be at least 3 µm, preferably 5 to 10 µm. the pores on the opposite upper side of the membrane should be not larger than 1 µm. Pores having a diameter of 0.1 to 0.5 µm are preferred.

The special pore structure of these membrane matrices is evidently of decisive significance for the detection elements according to the invention. As shown by SEM photographs the mechanisms of these systems can be interpreted as follows. Whole blood flows unimpeded through the large pores (>5 µm) on the application side of the membrane while plasma separation takes place through the increasing pore narrowings in the active separating layer.

By contrast, low molecular compounds including the low molecular products of the detection reaction, such as hydrogen peroxide, can pass through the microporous active separating layer.

If, then, a chromogenic detection system is introduced into the active separating layer or onto its surface, a detection reaction without interference from erythrocytes can be observed at the membrane surface after application of blood onto the reverse side of the membrane.

It is also possible to use other detection methods, for example electrochemical detection methods, instead of a chromogenic detection system because membranes, as described in DE-OS (German Published Specification) 36 15 831, can be easily metallized and therefore made electrically conductive.

## Brief Description Of The Drawings

Other and further objects, advantages and features of the invention will be apparent to those skilled in the art from the following detailed description thereof, taken in conjunction with the accompanying drawings in which:

Fig. 1 is a diagrammatic cross-sectional view of a portion of a reagent matrix membrane usable in accordance with the present invention;

Fig. 2(a) and (b) are a diagrammatic perspective view of a test strip in accordance with the present invention and a cross-sectional side view of the same test strip, respectively;

Fig. 3 is a side view of a test strip in accordance with another embodiment of the present invention;

Fig. 4(a) and (b) are diagrammatic side views of two other embodiments of the present invention;

Fig. 5 is a series of four drawings illustrating in sequence the preparation of the test strip embodiment

illustrated in Fig. 3 [Figs. 5(a) through (c)] and in Fig. 4(a) [Fig.5d];

Fig. 6 is a side view of yet another embodiment of the present invention;

Fig. 7 is a diagrammatic perspective view of a test strip in accordance with the present invention in which a color chart is positioned on the test strip in the vicinity of the location where visual readings are made;

Fig. 8 is a series of five drawings which illustrates the preparation of yet another test strip in accordance with the present invention by sequential illustrations (a) through (d), which lead to the preparation of the embodiment shown in side view Fig. 8e; and

Figs. 9 to 11 are diagrammatic side views of three additional embodiments of the present invention, all of which contain an opening in the substrate beneath the reagent matrix for visualization of the color change which occurs in the reagent matrix following application of sample to the test strip.

In the figures the last digit of an item number is identical for similar parts in different figures.

## Description Of The Preferred Embodiments

An important advantage of the detection elements described here is the relative ease with which they can be prepared. For example, the membrane systems shown in Figure 1 can be prepared according to the present state-of-the-art in a single operation (coating a carrier material with a polymer casting solution followed by coagulation and drying). In the diagrammatic representation of the membrane in Figure 1(A) is the active separating layer which usually has a thickness of 0.2 $\mu$m to 2.9 $\mu$m; (B) is the integrally asymmetric polymer membrane which is normally 50 $\mu$m thick; and (C) is a layer of material which can be a fabric or a wadding, the thickness of which is about 10 to 250 $\mu$m.

The reagents necessary for the detection reaction can be incorporated, as described in detail in DOS (German Published Specification) 34 07 359, in the membrane either directly via the polymer casting solution or by subsequent impregnation. The latter is preferred in the case of water soluble reagents. A combination of these two methods is used in many cases, organic soluble reagents being incorporated via the polymer casting solution and water soluble substances, such as enzymes, being incorporated by impregnation. Impregnation is preferably carried out with the extruder or cascade method described in EP-A 246 505.

A further advantage of the detection elements described here is the wide selection of possible polymers. Virtually all soluble polymers are suitable for preparation of the membrane by precipitation coagulation, also called phase inversion. Thus, there is a selection of hydrophilic, hydrophobic, neutral, cationic and anionic polymers which allows adjustment to the particular detection system.

Examples of suitable polymers include: polyvinylidene fluoride, polysulphone, polyhydantoins, styrene/maleic anhydride copolymer, polyamides, cellulose acetates, polyethers, polycarbonates, polyurethanes, where appropriate in combination with ionic polyurethane dispersions, polyacrylonitrile and copolymers thereof. Acrylonitrile copolymers with ionic, in particular cationic, groups are preferred.

Acrylonitrile copolymers with anionic functions, betaine structures or mixtures of cationically and anionically modified copolymers can also be employed. Example 1 shows the chemical structure of a preferred cationic acrylonitrile polymer.

The acrylonitrile copolymers contain at least 50 percent by weight, preferably at least 80 percent by weight, of acrylonitrile.

Suitable copolymerized comonomers are both neutral comonomers and, as mentioned, comonomers having anionic, cationic or betaine functions.

Examples of neutral monomers which can be present in a polymer, alone or in combination with one another, in amounts of 0.5 to 50 percent by weight, are, along others, methacrylonitrile; (meth-)acrylic esters, e.g., methyl, ethyl, butyl, hexyl, 2-ethylhexyl, cyclohexy, hydroxyethyl, hydroxypropyl, ethoxyethyl, butoxyethyl, methoxyethyl, phenyl, phenylethyl, phenylpropyl, furfuryl, tetrahydrofurfuryl, polyalkylene ether (meth)acrylate; vinyl esters such as vinyl formate, vinyl acetate, vinyl propioniate, vinyl butyrate, vinyl benzoate; (meth-)acrylamide and N-mono- or N,N-dialkyl- or hydroxy- and alkoxyalkyl derivatives thereof, e.g., dimethyl(meth)acrylamide, N-hydroxymethyl-, N-methoxymethyl(meth)acrylamide; maleic acid amide; itaconic acid amide; unsaturated ketones such as methyl vinyl ketone, phenyl vinyl ketone, methyl isopropenyl ketone; diacetone acrylamide; vinyl ethers such as vinyl methyl ether, vinyl ethyl ether; N-vinylcarboxyamides such as N-vinylformamide, N-vinylacetamide, N-vinyl-N-methylforamide, N-vinyl-N-methylacetamide; N-vinyllactams such as N-vinyl-pyrrolidone, N-vinylcaprolactam; styrene; $\alpha$-methylstyrene; and diisobutene.

Suitable anionic monomers which can be polymerized with acrylonitrile alone or in combination with

neutral monomers are unsaturated carboxylic acids, e.g., (meth)acrylic acid, itaconic acid, maleic acid, fumaric acid and the corresponding salts. The maximum content of free carboxylic acid in the polymer can be 2100 mEq/kg (milliequivalents per kilogram) of polymer. Other anionic monomers are unsaturated sulphonic acids and salts thereof, e.g., vinylsulphonic acid, (meth)allylsulphonic acid, styrenesulphonic acid, 2-acrylamido-2-methylpropane-sulphonic acid, potassium 3-sulphopropyl(meth)-acrylate, dipotassium bis(3-sulphopropyl)itaconate, (meth)allyloxybenzenesulphonic acid, sodium 2 sulphoethyl-$\alpha$-methylacrylate, potassium monolaurylitaconoxypropanesulphonate, and sodium sulphopropylalkylmaleate. The maximum content of free sulphonic acid functions in the polymer can be 1400 mEq/kg of polymer.

Cationic monomers are in particular those containing an amine or ammonium structure. They can also be incorporated in the polymer alone or in conjunction with neutral monomers. Examples are, among others: vinylpyridine; 2-methyl-5-vinyl-pyridine; N-mono- or N,N-dialkylaminoalkyl (meth-acrylates, e.g., N-tert-butylaminoethyl (meth)-acrylate, N,N-dimethyl-, N,N-diethyl-, N,N-dipropylaminoethyl (meth)acrylate, N,N-dimethylaminopropyl, -butyl, -pentyl, neopentyl, -hexyl(meth)acrylate; N-monoalkyl- and N,N-dialkyl-aminoalkyl(meth)acrylamide, e.g., N,N-dimethylaminopropyl(meth)acrylamide; N-vinylimidazole and derivatives, e.g., N-vinyl-2-methyl-, N-vinyl-2-ethyl-, N-vinyl-2-propyl-, N-vinyl-2 -isopropyl-, N-vinyl-4-methyl-, N-vinyl-5-methylimidazole, N-vinylimidazoline and derivatives, e.g., N-vinyl-2-phenyl-imidazoline; 1-($\beta$-methacryloxyalkyl)imidazoles such as 1-($\beta$-methacryloxyethyl)-2-methylimidazole, 1-($\beta$-methacryloxypropyl)-2-methylimidazole and 1-($\beta$-methacryloxybutyl)-2-methylimidazole.

These amine monomers can be present in the copolymer in free amine form or in the ammonium form either protonated by acids or quaternized by alkylating agents. Based on free amine, the polymer can possess a maximum of 1600 mEq of basic groups per kg of polymer.

A further group of functional monomers, up to 15 percent by weight of which can be contained in the acrylonitrile copolymer, have betaine structures, i.e., they are inner salts. Examples include: N-(3-sulphopropyl)-N-methacryloxethyl-N,N-dimethylammonium betaine, N-(3-sulphopropyl)-N-methacrylamidopropyl-N,N-dimethylammonium betaine, and 1-(3sulphopropyl)-2-vinyl-pyridinium betaine.

The acrylonitrile copolymers mentioned are prepared by known methods of precipitation, emulsion or solution polymerization with the aid of free radical-forming initiators, e.g., azo compounds, peroxides, redox systems such as persulphate/sulphite, persulphate/thiosulphate, persulphate/chlorate, and furthermore hydrogen peroxide/sulphinic acid.

For synthesis of polymers containing cationic or betaine comonomers the preferred starter system is, if precipitation polymerization in an aqueous medium is employed, hydrogen peroxide/mercaptan, as described in DP 2 046 086.

The molecular weights of the acrylonitrile (co)polymers, expressed as K value (according to Fikentscher, Cellulosechemie 13, 58 (1932), are 70 to 130.

Depending on the polymer the following solvents are used for preparation of the casting solution:

Dimethylformamide, N-methylpyrrolidone, dimethyl sulphoxide, dimethylacetamide, formamide, glycol carbonate, acetone and mixtures thereof. Besides the known organic solvents and inorganic acids and aqueous salt solutions. Particularly suitable solvents for the preferred polyacrylonitriles are dimethylformamide, dimethyl sulphoxide and formamide and mixtures thereof.

Where appropriate, small amounts of nonsolvents, for example, alcohols or water, can be added to the polymer casting solutions.

Preferred coagulation liquids are aqueous systems, in particular pure water or water containing a small amount of solvent, for example, 95 percent by weight of water, 5 percent by weight of dimethylformamide. The membrane is advantageously washed with pure water before drying.

As mentioned, and generally known, the casting solutions employed for the preparation of the membranes conventionally consist of a polymer or mixture of polymers and a solvent or mixture of solvents. Fillers can be added, where appropriate, for stabilization of the membrane structure and for adjustment of certain reflection properties, so that filler-containing membranes are obtained.

It has now been found, surprisingly, that such membranes prepared in accordance with the standard formulations are unsuitable for the preparation of the no-wipe blood glucose test strips, according to the invention, with tetramethylbenzidine as an oxidation indicator.

As described in detail in the comparison example, the customary TMB/GOD/POD system for glucose detection was incorporated in a polyacrylonitrile membrane and the test was carried out via the membrane carrier side with aqueous glucose standard solutions and whole blood. Very weak violet-gray colorations which hardly allowed differentiation and displayed very poor color stability were obtained.

Surprisingly, these deficiencies were completely eliminated by addition of certain anionic surfactants, for example sodium dodecylbenzenesulphonate, to the polymer casting solution, blue colorations with good color gradations and excellent color stability being obtained. Sodium dodecyl sulphate, lithium dodecyl

sulphate, tris(hydroxymethyl)aminomethane lauryl sulphate, sodium dioctylsulphosuccinate and sodium dodecanesulphonate proved suitable in addition to sodium dodecylbenzene sulphonate. By contrast, membranes to the casting solution in which the sodium salts of pentanesulphonic acid, heptanesulphonic acid or octanesulphonic acid were added and nonionic surfactants such as Triton X100 (ethoxylated nonylphenol) demonstrated poor results as membranes without added surfactants.

Beside the type of the surfactant used, the amount also plays an important role. For example, addition of 4.3 percent by weight of sodium dodecylbenzene-sulphonate produced an improvement in color gradation as compared to the surfactant free formulation but the color stability was not satisfactory. increasing amounts of the anionic surfactant increasingly improved the color gradations and color stabilities, the optimum being at about 8 to 25 percent by weight, based on polymer solid, equivalent to surfactant addition of about 1 to 4 percent by weight, based on polymer casting solution.

For reflectometric evaluations it is frequently favorable, as described in DOS (German Published Specification) 34 07 359, to improve the reflection properties of the reagent membrane by incorporation of fillers, such as $TiO_2$, $BaSO_4$, $SiO_2$, talc, $CaCO_3$, zeolites, bentonites, diatomaceous earth or microcrystalline cellulose. Detection elements with defined primary colors can be obtained by means of color pigments. In the preparation of filler containing detection elements for the range of applications according to the invention it was found, surprisingly, that the kinetic course of the detection reaction is sensitively dependent on the filler content in the membrane. Thus, filler free membranes showed a typical "kinetic" course of the reaction, for example in the glucose detection, whereas membranes having certain filler contents gave an end-point reaction. A comparison of Examples 1 and 3 illustrates this surprising result.

For continuous preparation and for better handling, precipitation coagulation membranes are preferably cast on membrane carrier materials. The finished membrane is then a composite of semipermeable membrane with the carrier material. Suitable carrier materials are known to membrane experts and consist, for example, of polymer waddings, multifilament polymer fabrics or multifilament glass fiber fabrics. For reasons of better homogeneity, fabrics are preferred for the detection elements according to the invention, for example polyester fabric PES 1973 or nylon fabric PA 1153 (Messrs. Verseidag, Krefeld, Schweiz. Seidengazefabrik, Thal, Switzerland) and glass fiber fabric type 03249 (Messrs. Interglas, Ulm).

In contrast to the "test strips on multifilament fabric carrier layers" described in European Patent Specification 0 113 896, the detection elements described in the present application are completely able to function, even without carrier material, with respect of erythrocyte separation and color reaction. The carriers primarily serve for improved continuous preparation and easier handling during packaging to give ready-made test strips.

In the simplest case, the detection elements according to the invention can be converted into ready-made test strips (10) in the manner described in Figure 2. On to a preferably white perforated polymer film (1), for example Trycite® polystyrene film, the reagent matrix is attached at its carrier site by means of a double-sided adhesive tape (3). The polymer film (1) serves as a test strip support. Whole blood is applied above the round opening (4) in polymer film (1). The color reaction and its evaluation take place on the opposite side of the membrane matrix (membrane surface) which is available for direct access of oxygen and which therefore does not require any additional aids to supply oxygen.

It has now been found, surprisingly, that detection elements can be provided with further advantages as compared to the conventional system by a novel test strip (20) structure, as shown in Figure 3. Figure 3 shows a version of this test strip structure according to the invention, the preparation of which is described stepwise in Figures 5(a) to (c). The reagent matrix (12) is attached with its active separating layer (reagent layer) onto a transparent polymer film (11) by means of double-sided adhesive tape (13). After application of the sample liquid the color reaction can be observed through the transparent polymer film (11) which serves as both test strip support and window. The atmospheric oxygen necessary for the reaction is stored in the free air space (16).

As shown in Figure 4a, one or more permeable layers (48), such as waddings of polymer or glass, paper or a fabric of polymer or glass, can be attached to the top side of the reagent matrix (42) to improve distribution and adsorption of the blood sample, and these can in turn be covered, where appropriate, by a polymer film (49) except for opening (47). The preparation of test strip (50) is thus based on the steps taken to prepare test strip (20) of Fig. 3 and this is illustrated in Fig. 5 (a) to (d). In this way, it is also possible to prepare test strip (60) (shown in Fig. 4b) with several reagent matrices (52) to which blood can be applied, where appropriate, via an opening (57).

As shown in Figure 6 the transparent test strip support can be replaced by a nontransparent polymer film (21) having a transparent window (28). Accordingly, the transparent "window" (28) and the free air space (26) between window and reagent matrix (22) are essential to the invention of the novel test strip structure (30). The volume of the free air space (26) depends on the amount of oxygen required for the

reaction and can be made variable by the thickness of the double-sided adhesive tapes (23) and the polymer films. As can be easily calculated, the conventional substrate concentrations are sufficient for the oxygen requirement for diagnosis reactions when conventional double-sided adhesive tapes or films are used.

It has been found that in test strips having such a structure the autoxidation reaction of the oxidation indicator catalyzed by air and light process much more slowly than in test strips having a conventional structure. This positive effect can be further intensified by transparent windows with UV absorbers.

As shown in Figure 7, corresponding color blocks (37) allowing better visual evaluation can be integrated, where appropriate, in or below the transparent window or opening (38), attached to substrate (31) as shown on test strip (40).

In the detection elements according to the invention the individual layers can be fixed either with double-sided adhesive tape (13, 43, 53) or liquid adhesive or by hot-melt adhesives, for example EVA hot-melt adhesives from the 3M company. Hot-melt adhesives are partioularly preferable for attachment of polymer networks with the test strip support.

Furthermore, it has been found, completely surprisingly, that the detection systems according to the invention having the test strip structure just described can also be employed in an outstanding manner for dip-and-read tests for investigation of urine or other aqueous samples. As described in detail in EP-A 64 710 and DE-OS (German Published Specification) 38 09 523.8, the previously known microporous polymer matrices cannot be employed directly without further aids for dip-and-read tests because their surfaces wet nonuniformly and absorb liquid too slowly. By contrast, with the test strip structure described in the present application access of liquid via the microporous active separating layer (reagent zone) is impossible.

In urine test strips the top cover (49) in Figure 4a (preferred for blood test strips) is preferably missing.

Yet another embodiment of the invention is illustrated in Fig. 6 where test device (30) consists of a substrate (21) onto which reagent matrix (22) is affixed by means of double-backed adhesive (23) on one side thereof and on the other side thereof transparent plastic member (28) is affixed by means of double-backed adhesive (23). An air space (26) is present between the matrix (22) and the transparent plastic material (28). The transparent plastic material (28) permits readings to be made from the bottom of test device (30).

The test devices can, if desired, contain color charts surrounding the area for visual reading. An example of this is illustrated in Fig. 7 where test strip (40) contains color charts (37) applied to one side of the substrate (31) surrounding opening (38). The color elements (37) can, if desired, contain numerical values to aid in the determination of the readings being made. In Fig. 7 the color chart areas (37) contain numerical values ranging from 50 to 500.

A proposal for the stepwise construction of a urine test strip (70) is shown in Figure 8(a) to (e). The components in Figure 8 are designated as follows: Reagent matrix (62), double-sided adhesive tape (63), transparent polymer film (61), polymer network and absorbent material (68), and free air space (66).

With regard to liquid residues which remain adhering to the test strip after immersion (drop problem) it is frequently advantageous to provide a test strip (70) in which the absorbent material (68) is not applied completely down to the end of support (61) with adhesive tape (63); as shown in the cross-sectional representation in Figure 8(e). Where appropriate, opposite edges of the test strip support (61) can be left free of double-sided adhesive tape (63).

The reagent matrix of the dip-and-read test strips with the test strip structure shown here can also consist, where appropriate, of paper, although plastic matrices are preferred.

Since the reagents necessary for the reaction can be incorporated in different layers (active separating layer, matrix polymer, membrane carrier material and overlying absorptive layer or layers) in such detection elements one skilled in the field is provided with a number of further possible combinations.

Diagnosis test strips with covering network layers have been described in DE-OS (German Published Specification) 31 18 381; however, the covering network layer in this specification is arranged directly above the reagent layer. Any auxiliary reagents are in direct contact with the reagent layer and on application of sample are easily carried into the reaction zone lying immediately underneath. Therefore, as described in DE-OS (German Published Specification) 30 12 368, it is possible to incorporate only those reagents which cannot undergo any undesirable secondary reactions with the reagent system in the reagent matrix. For example, the only oxidizing agents which can be used for solving the problem of ascorbic acid interference are those which are incapable of entering into a reaction with the oxidation indicator (usually tetramethylbenzidine).

Since the color reaction in the systems described in DE-OS (German Published Specification) 31 18 381 can be evaluated only through network coverings, these must meet special requirements which greatly limit the choice of reagents, materials and mechanisms which can be used.

For example, it is possible to use only transparent polymer fabrics, and incorporation of auxiliary reagents into these fabrics is limited to colorless products. A further disadvantage of detection systems with networks on the evaluation side is the difficulty of reflectometric evaluations which are frequently rendered incorrect by the reflection properties of the polymer network.

The limitation with respect to materials and type of incorporated reagents do not apply to the permeable layers (68) described for test strip (70), or layer (48) for test strip (50), or layer (58) for test strip (60). Rather, the corresponding materials can be adjusted to fulfill the particular task. For example, thin fabrics or waddings having a good distribution function for blood (for example nylon fabric Nytal® and Nytal XXX® of the Schweizerische Seidengazefabrik or Paratex® waddings from Lohmenn) are preferred for blood test strips, in particular with several test zones (Figure 4b). By contrast, materials having a higher absorptive capacity, for example Viledon Filter FFM 2687®, Paraprint® waddings, glass fiber papers or polyester or nylon fabrics with basis weights of about 50-100 g/m$^2$ (grams per square meter), are more suitable for test strips to be used by the dip-and-read method. Other suitable materials for the permeable layers are filter papers having basis weights of 9.5 g/m$^2$ to 27.5 g/m$^2$ which are prepared, for example, by Schoeller and Hoesch for various filtration purposes.

In the case of test strips for dip-and-read tests it is occasionally advantageous to interpose a layer with absorptive material which is capable of absorbing defined liquid amounts between the polymer network and the reverse side of the reagent matrix. Preferred materials for this purpose are waddings or fabrics having defined absorption capacities for liquid; particularly preferably laminates with water-absorbing polymers, for example laminates from Gelok Int. Corp.

On incorporation of reagents in one or more of these layers there is no need to take into account the limitations mentioned in respect of interaction with the reagents of the reagent layer.

Further test strip structures, according to the invention, for blood analyses are shown in Figures 9 to 11. These test strips correspond to those in Figures 3 and 4, the transparent carrier film (11, 41, 51) being replaced by a white polymer film (71, 81, 91). This test strip configuration is preferred for blood analysis over the type shown in Figures 3 and 4 whenever the corresponding indicator system is not photosensitive.

The wadding, fabric and film materials selected from the test strip structure are essential to the optimum course of the detection reaction and are described in somewhat greater detail below.

## Waddings

Messrs. Freudenberg offer special waddings of polypropylene and polyester in various thicknesses and basis weights for preparation of membranes. These waddings have proved suitable, for example, as membrane carrier material (C in Figure 1). The "waddings for liquid filtration" of Messrs. Freudenberg are also suitable. These are predomi nantly waddings of cellulose with synthetic resin polymer as binder which have proved suitable as the absorptive layer (58) in Figure 4b and 68 in Figure 8e.

Messrs. Lohman offers waddings based on cellulose, predominantly for hygienic and medical applications. These articles have also proved suitable for the same absorptive layer. Laminates of waddings with water-absorbing polymers absorbing defined amounts of liquid are offered, for example, by Messrs. Gelok. Such laminates have proved suitable, in particular, for dip-and-read tests and blood diagnosis tests in respect of liquid dosing. Similar properties were able to be achieved with glass fiber waddings (Messrs. Binzer). Test strips with liquid-dosing layers are frequently advantageous with regard to end-point reactions.

## Fabrics

Fabrics of nylons, polyester, polyacrylonitrile, fluorinated polymers, cotton and glass fibers in different weaves, thread thicknesses, open areas and basis weight are offered by various companies (for example Verseidag, Züricher Beuteltuchfabrik AG (ZBF), Interglas, Schweizerische Seidengazefabrik). Depending on the consistency, these fabrics can be used either as membrane carrier material (C in Figure 1) or as the permeable layers (48, 58, 68, 88, 98). Fabrics having basis weights of about 100 to 350 g/m$^2$ (for example, PES 1973 from Verseidag, polyester 2F/777 from Schweizerische Seidengazefabrik or the glass fiber fabric 03249 from Interglas) have proved suitable, for example, as membrane carrier material. Fabrics having basis weights of about 100 to 200 g/m$^2$ showed a good distribution function for blood in blood diagnosis test strips, in particular with several reagent fields. Messrs. Schweizerische Seidengazefabrik also offer metallized fabrics which can detect, where appropriate, the starting point of the sample application via measurement of the ion conductivity.

Films

White-pigment films (for example Trycite® polystyrene film) are preferably used for preparation of the test strip supports. For transparent polymer films which also serve as windows, films of very perfect transparency are preferred, for example films of cellulose acetate (Ultraphan®), polycarbonate (Makrofol®), polyethylene terephthalate (Melinex®) or fluorinated polymers (PVDG, Messrs. Lonza). Hydrophobic or hydrophobized films (e.g., silicone-treated films from Laufenberg, Krefeld) are preferred for dip-and-read tests.

Other suitable materials which can be used as permeable layers are microfilter membranes, for example nylon membranes from Pall or membranes of fluorinated polymers from Millipore.

The membrane matrices, according to the invention, having an asymmetric pore structure which preferably adhere to waddings or fabric carriers, permit selective adjustment, in particular in combination with the test strip configurations according to the invention described, of specific system properties which are not yet known in this range of variations and combination possibilities from previously known test strip systems. The decisive influencing factors are initially to be briefly explained here and will be described in greater detail in the subsequent examples.

Plasma separation inherent to the system

The decisive influencing factor is the already mentioned asymmetric pore structure. As was demonstrated by electron microscopy plasma separation takes place at the boundary layer between the highly porous support membrane and active separating layer.

Preceding part-reaction

The necessary reagents are incorporated on the reverse side of the membrane, preferably in the membrane carrier material (C in Figure 1), particularly preferably in the overlying polymer network or absorptive material.

**Example - Ascorbic Acid Interference**

A test strip according to Figure 8e is prepared, the reagent matrix (62) containing the reagents necessary for detection of glucose. The membrane carrier material or the polymer network or absorptive material (68) lying over the matrix is provided with the reagents selective for ascorbic acid. As described in DE-AS (German Published Specification) 15 98 008, these can be compounds with anion exchanger functions or preferably compounds having oxidizing properties as described, for example, in DE-OS (German Published Specification) 30 12 368. However, when choosing the reagents, the limitations described in the lastmentioned application with respect to interaction of oxidizing agent with the indicator are not necessary here because the detection elements according to the invention permit spatial separation from the reagent layer. The specific structure of the detection elements makes it furthermore possible to integrate compounds with different function principles, for example, with anion exchanger and oxidation function in one or more layers in front of the reagent matrix in order to remove interfering components. Possible reagents for selective reaction with ascorbic acid which may be mentioned are: $Na_5IO_6$ and Pb $(OAc)_2$ (DE-OS [German Published Specification] 35 39 772); p-diazobenzenesulphonic acid, $H_3PO_3$ and $NaIO_4$ or $CuSO_4$ (European Patent 160 239); iron complexes or hydroperoxides (European Patent Application 123 115) or ascorbic acid oxidase (DE 29 07 628).

Effect on reaction kinetics

It has been found, completely surprisingly, that the detection elements according to the invention can be used for end-point and kinetic determinations by simple modification of the polymer casting solution used for the preparation of the membranes. As was surprisingly found and is described in detail in Examples 1 and 3, filler-free membrane matrices lead to a kinetic reaction, whereas filter containing membranes show a typical end-point reaction.

It was furthermore surprisingly found that a delay in the start of the reaction can be adjusted in the detection elements according to the invention. Such a course of the reaction is occasionally advantageous, for example in the case of preceding reactions or in view of the exact timing between application of sample and measurement. This surprising result was observed on preparation of glucose test strips in which the reagent layer consisted of an aqueous emulsion containing TMB, GOD, POD and citrate buffer. Polyvinylpyrrolidone was used as emulsifier. Other water-soluble polymers suitable for this purpose are polyethers, polyacrylic and polyvinyl compounds as well as natural water-soluble polymers, such as gelatin, agarose or cellulose derivatives, where appropriate in combination with ionic or nonionic surfactants. The preparation of such aqueous TMB emulsions is described in Example 6. The use of aqueous TMB emulsions for the preparation of reagent layers has not yet been disclosed.

The absorption properties of an additional layer (48, 58, etc.) can also be used to influence the sensitivities of the chromogenic reaction. Thus, increasing color intensities were observed with papers having increasing basis weights (in the range of 9.5 $g/m^2$ to 27.5 $g/m^2$).

Use for dip-and-read methods

Owing to the test strip structure already described and shown in Figure 1, the detection elements according to the invention can also be used for dip-and-read methods. By using specific laminates, waddings or fabrics on the reverse side of the matrix the problem of supernatant residual liquid can be solved and the amounts of absorbed liquid controlled.

The use of the detection elements, according to the invention, for test strips in which the sample liquid is applied to the reagent layer side and the excess is wiped off is, or course, also possible with a conventional test strip structure.

A further advantage of the detection elements according to the invention as compared to test strips with glass fiber waddings for separation of erythrocytes is the very small amount of liquid necessary for the test. On the one hand, this is advantageous for the patients in the case of blood analysis permitting relatively small amounts of sample to be applied to several test fields. This provides the possibility, shown in Figure 4b for example, that several test fields can be operated by application of a relatively small amount of sample to one point (opening 57) in the detection system. Thus, several metabolites can be detected simultaneously, or color reactions in different color shades and graded sensitivities with respect to better color differentiation can be generated within one metabolite (e.g., glucose) by modification of the reagent system.

The following examples are intended to illustrate the invention further.

## EXAMPLE 1

Preparation of a glucose detection system (reaction color: blue).

(a) Preparation of the Polymer Casting Solution

A homogeneous solution was prepared by stirring from 146.9 g (grams) of a cationic acrylonitrile copolymer, 37.4 g of dodecylbenzenesulphonate (DBS), 803.4 g of dimethylformamide (DMF) and 12.3 g of 3,3′5,5′-tetramethylbenzidine (TMB).

The chemical structure of a cationic acrylonitrile copolymer was

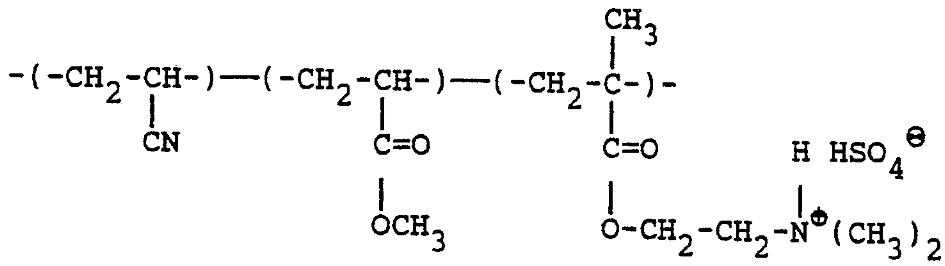

89.4% 4.9% 5.7%

K value: 84

Amine functions: 229 mEq/kg polymer

After filtration (Seitz filter KO 00) and degassing, a polyester fabric was knife-coated, coagulated in pure water and subsequently dried in hot air.

Test conditions:

Wet application of knife-coating: 150 µm

Polyester fabric: PES 1973 (verseidag), 120 g/m$^2$, 170 µm thick

Coagulation conditions: pure water

Water temperature: 45 °C, 3 minutes

Drying: hot air, 3 minutes at 65°.

(b) Enzyme Impregnation

An aqueous impregnation solution was prepared from glucose oxidase (GOD), peroxidase (POD), Triton X100, polyvinylpyrrolidone (PVPK 30) and citrate buffer (pH 5.5) (TL2).

The TMB-containing membrane matrix described in Example 1(a) was coated with impregnation solution on the front side of the membrane by means of a doctor blade (10 µm) and dried in a circulating drying cabinet for 3 minutes at 50 °C.

Test results:

(a) With whole blood - one drop of fresh whole blood was applied to the reverse side of the membrane (polyester fabric). A homogeneous blue coloration was observed after about 20 seconds on the opposite side (front side of the membrane).

(b) With glucose standard solutions (50, 100, 250, 400, 600 mg/dl), an increasing intensity in the blue coloration corresponding to the increasing glucose concentrations was observed approximately 10 seconds after application of the sample.

(c) Evaluation of the reaction kinetics with a reflectometer reveals very good concentration dependence.

## COMPARISON EXAMPLE 1

(Reagent membrane without anionic surfactant)

Polymer casting solution: 146.9 g of the cationic acrylonitrile copolymer, 803.4 g of dimethylformamide (DMF) and 12.3 g of 3,3′,5,5′-tetramethylbenzidine (TMB).

The further processing (membrane preparation, enzyme impregnation) was analogous to that in Example 1.

Test results:

Very slight violet-grey discoloration were found with the aqueous standard glucose solutions, and these

did not permit color differentiation corresponding to the glucose concentration. The reaction colors faded away after only a few minutes. No color reaction was observed with whole blood.

## EXAMPLE 2

Preparation of the system described in Example 1 in carrier-free form.

All test conditions and test results were identical, with the exception of the coating substrate. A glass plate was coated instead of the polyester fabric and was immersed in water (coagulated). During the coagulation process the membrane detached from the glass plate (carrier-free membrane). After drying and enzyme impregnation the test was carried out with whole blood and aqueous standard solutions analogously to Example 1.

In the case of whole blood, a color reaction without erythrocyte interference was observed on the front side of the membrane. Increasing color intensities were obtained with increasing glucose concentrations.

## EXAMPLE 3

Preparation of a glucose detection system with end-point reaction (reaction color: green).

A homogeneous dispersion was prepared by means of a high speed stirrer from 115.3 g of the cationic acrylonitrile copolymer from Example 1, 163.3 g of barium sulphate (Blac fixe micron), 29.3 g of DBS, 682.3 g of DMF, 0.1 g of Makrolex yellow G, and 0.7 g of TMB. Filtration (mesh width 25 $\mu$m) and degassing were followed by the further preparation steps as described in Example 1.

On testing with whole blood a homogeneous green coloration was observed approximately 10 seconds after application of the sample.

On evaluation of the front side of the membrane with a reflectometer, attainment of the end point was observed after approximately 2 minutes.

## EXAMPLE 4

Preparation of a glucose detection system (reaction color: red).

A homogeneous solution was prepared by stirring from 146.9 g of the cationic acrylonitrile copolymer from Example 1, 37.4 g of DBS, 803.4 g of DMF, and 12.3 g of 2,4,6-tribomophenol. The further steps up to the preparation of the dry, tribromophenol-containing membrane were analogous to Example 1.

An aqueous solution of GOD, POD, saponin, 4-amino-antipyrine (4-AAP), phosphate buffer (pH 5.5) and PVPK 30 were used for enzyme impregnation on the front side of the membrane (TL4).

After application of whole blood to the reverse side of the membrane a color reaction without erythrocyte interference was found on the front side of the membrane. The test solutions with increasing glucose concentrations showed increasing intensities in the reaction colors.

## EXAMPLE 5

Preparation of a glucose detection system with reduced reactivity in the low glucose range.

The glucose detection system described in Example 4 was additionally impregnated on the reverse

side of the membrane (polyester fabric) with an impregnation solution of GOD, POD, saponin, 4-AAP and phosphate buffer (TL3) and dried.

In the test with the aqueous standard solutions (sample application to the reverse side of the membrane), a color reaction was observed only from 400 mg/dl of glucose onwards, and this was increasingly intense with increasing glucose concentration.

## EXAMPLE 6

Preparation of a glucose detection system with delayed reaction start.

A reagent-free membrane was prepared in analogy to Example 1 from 146.9 g of the cationic acrylonitrile copolymer, 37.4 g of DBS, 803.4 g of DMF, and 12.3 g of 2,4,6-tribomophenol. For impregnation, an aqueous TMB emulsion was prepared with the aid of a bead mill under the following conditions: 300.0 g of a 20% strength PVP solution in citrate buffer (0.2 M, pH 5.5), 15.0 g of TMB and 0.75 g of $NaBH_4$ were milled for 25 minutes with the aid of 900 g of glass beads and then filtered. 180 mg of GOD (180 U/mg [units per milligram]), 1440 mg of POD (50 U/mg) and 30 ml of citrate buffer (0.2 M, pH 5.5) are incorporated with the aid of a magnetic stirrer into 50 ml (milliliters) of this suspension.

After degassing, this reagent emulsion is coated onto the surface of the reagent-free membrane and dried.

Test with whole blood and aqueous glucose standard solutions:

In the test with whole blood, a color reaction without erythrocyte interference was observed after approximately 40 seconds.

With the aqueous glucose standard solutions, increasing color intensities corresponding to the concentration were observed approximately 30 seconds after application of the sample.

In further comparison examples, the following membranes were used instead of the cationic acrylonitrile copolymer membrane:

(a) Polyamide/$TiO_2$ membrane

A polymer/filler dispersion was prepared with the aid of dissolver from 100.0 g of polyamide (Durethan T 40®), 650.0 g of DMF and 566.7 g of $TiO_2$. The membrane was prepared in analogy to Example 1.

(b) Polyhydantoin/$TiO_2$ membrane

A dispersion was prepared from 100.0 g of polyhydantoin, 88.90 g of NMP and 566.7 of $TiO_2$, and subsequently a membrane was prepared in analogy to Example 1.

(c) Polyether-polycarbonate membrane

A homogeneous casting solution was prepared from 83.0 g of dioxolane and 17.0 g of polyether carbonate (KU 1-1013, Bayer AG) and subsequently a membrane was prepared in analogy to Example 1.

(d) Polysulphone membrane (Tu-AN 4840 420, Kalle)

In analogy to the cationic acrylonitrile copolymer membrane described, the membranes were coated with the above reagent emulsion, dried and tested with whole blood and aqueous glucose standard solutions.

The test results (color reaction) corresponded to those of the above acrylonitrile copolymer membrane.

## EXAMPLE 7

Test strips for the detection of glucose in urine.

A test strip according to Figure 8 was prepared, the reagent matrix described in Example 1 being used. The permeable layer (68) used was a nylon fabric (PA 15/10, Nybolt, from Schweizerische Seidengazefabrik AG, Zürich). After immersion in glucose-containing urine and withdrawal the test strip was free of excess urine. A homogeneous blue coloration was observed through the transparent window. Increasing glucose concentrations led to increasing color intensities.

## EXAMPLE 8 .

Test strips for the detection of ketone in urine.

The reagent-free membrane described in Example 6 was impregnated on its reverse side with the following solution and dried: 4.0 g of sodium nitroprusside, 20.0 mg of water and 16.4 of magnesium sulphate ($MgSO_4$ 7 $H_2O$).

The pH value was adjusted to 9.4 with concentrated sodium hydroxide solution. Acetoacetic acid solutions (5, 15, 40, 80 and 160 mg/dl [milligrams per deciliter]) were used for testing, and these led to increasing color intensities corresponding to the concentration.

## EXAMPLE 9

Urine test strips with reduced ascorbic acid interference.

In analogy to Example 8 a glucose test strip was prepared, the permeable layer having been impregnated beforehand with a 3 percent strength sodium periodate ($NaIO_4$) solution. A solution of 200 mg/dl of glucose with 100 mg/dl of ascorbic acid was prepared for the test. After immersion a homogeneous color reaction having an intensity corresponding to that of the ascorbic acid-free comparison solution was observed through the transparent window.

No color reaction was observed in the comparison test strip without periodate impregnation.

## EXAMPLE 10

Detection of potassium in blood.

A homogeneous solution was prepared by stirring from 100.0 g of the acrylonitrile copolymer described, 25.5 g of lithium dodecyl sulphate, 692.6 g of dimethyl sulphoxide, 0.4 g of 7-(n-decyl)-2-methyl-4-(3,5-dichlorophen-4-one)-indonaphthol (7-decyl MEDPIN), 0.1 g of nitrophenyl octyl ether and 1.0 g of 2,3-naphtho-15-crown 5.

After filtration and degassing, a membrane was prepared on polyester fabric in analogy to Example 1.

Whole blood with increasing KC1 concentrations (0, 4, 8 and 12 mm) was used for the test. Blue colorations with increasing color intensities were observed on the membrane surface.

## EXAMPLE 11

Protein detection in urine.

The reagent-free membrane described in Example 6 was impregnated with the following solution: 0.024 g of tetrabromophenol blue, 40 ml of ethanol and 50 ml of citrate buffer (0.5 M, pH 3.3) make up to 100 ml with water. After drying, test strips were prepared analogously to Example 7 and immersed in albumin-containing standard solutions (0. to 500 mg/dl). Green colorations increasing with the concentration were observed with protein-containing solutions.

## EXAMPLE 12

Detection of urea in blood.

A homogeneous polymer solution was prepared by stirring, with heating, from 20.0 g of polyvinylidene fluoride (PVDF) and 100.0 g of N-methylpyrrolidone (NMP).

A hydrophobic membrane was prepared by coating of a polymer wadding and coagulation in water in analogy to Example 1. Drying was followed by impregnation with the following solutions:

| (a) Reverse side of the membrane | |
|---|---|
| Urease S<br>Hepes<br>DuPont Zonyl FSN | 150 U/ml Boehringer Mannheim<br>2.38 mg/ml Boehringer Mannheim<br>5 µl/ml nonionic fluorinated surfactant |
| (b) Front side of the membrane | |
| Bromophenol blue<br>Citric acid<br>DuPont Zonyl FSN | 20 mg/ml<br>2.1 mg/ml (0.01 M, pH 2.9)<br>5 µl/ml |

After drying, the test was carried out with urea-containing blood. A homogeneous blue coloration which became more intense with increasing amounts of urea was observed.

## EXAMPLE 13

Preparation of a glucose test strip with red reaction color in the low glucose concentration range and blue reaction color in the high glucose concentration range.

The tribromophenol-containing membrane described in Example 4 was impregnated from the reverse side with impregnation solution TL3 (GOD, POD, saponin, 4-AAP) and from the front side with the TMB-PVP emulsion described in Example 6, and was dried.

Preparation of the Impregnation Solution

A 50 cm (centimeter) graduated flask was used for the preparation of each of the solutions.

GOD (180 U/mg) POD (149 U/mg)

TL 1

119 mg GOD

335 mg POD

500 mg Triton X 10

Citrate buffer (pH 5.5, 0.2 M) up to the calibration mark

TL 2

2000 mg PVP K 30 in addition to TL 1

TL 1C

408 mg 4-aminoantipyrine in addition to TL 1
TL 2C
153 mg 4-aminoantipyrine in addition to TL 2
TL 3
119 mg GOD
335 mg POD
50 mg saponin
102 mg 4-aminoantipyrine
TL 4
2000 mg PVP K 30 in addition to TL 3

In a test with aqueous standard solutions a red reaction color was observed up to 100 mg/dl of glucose and a blue reaction color was observed at higher glucose concentrations.

From the foregoing, it will be seen that this invention is well adapted to attain all of the ends and objects hereinbefore set forth, together with other advantages which are obvious and inherent.

Obviously, many modifications and variations of the invention as hereinbefore set forth can be made without departing from the spirit and scope hereof and therefore only such limitations should be imposed as are indicated by the appending claims.

## Claims

1. A test strip for analyzing substances from biological fluids, which test strip consists essentially of
a porous membrane having asymmetric pore structure attached to a substrate, the porous membrane characterized by having a sample application surface of large pore size and an opposite surface of fine pore size adjacent to said substrate; and
wherein the membrane contains anionic surfactant in an amount of about 1 to about 4 percent by weight, based on a polymer casting solution used to form said membrane.
2. The test strip of claim 1 in which the asymmetric membrane has an active separation layer of 0.2 to 2.9 µm.
3. The test strip of claim 1 in which a sample application layer of 10 to 250 µm is applied to the membrane.
4. The test strip of claim 1 in which the porous membrane has a thickness of up to 50 µm.
5. The test strip of claim 1 in which the substrate is transparent.
6. The test strip of claim 1 in which an air space exists between a portion of the membrane and the substrate.
7. The test strip of claim 1 in which the strip is covered by a polymer film which has an opening for the introduction of a sample of the biological fluid being analyzed.

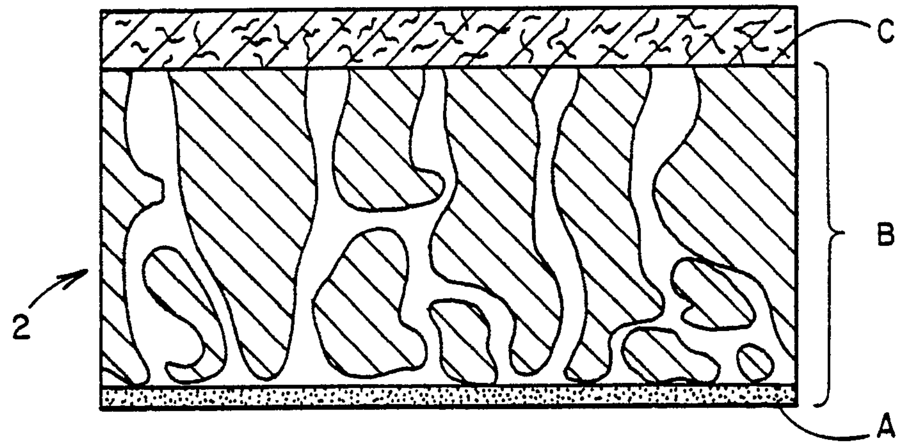


FIG. 1

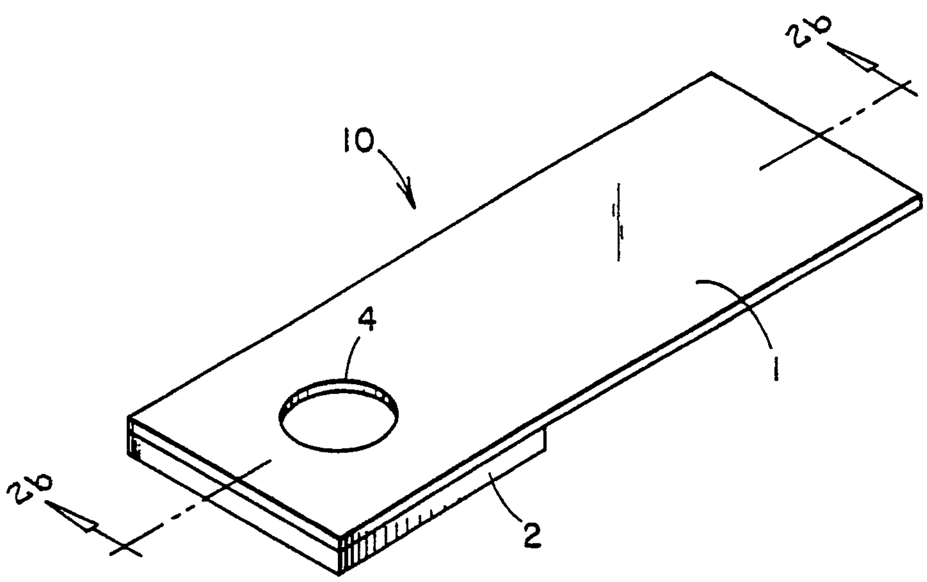


FIG. 2a

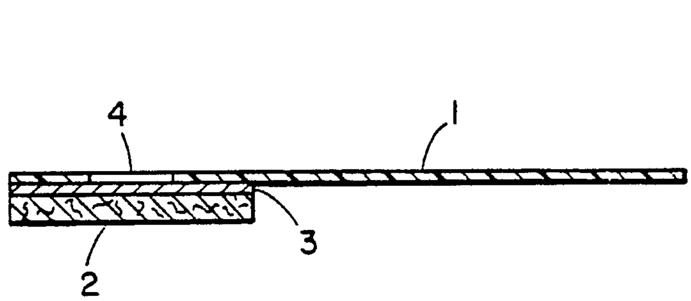


FIG. 2b

2/4

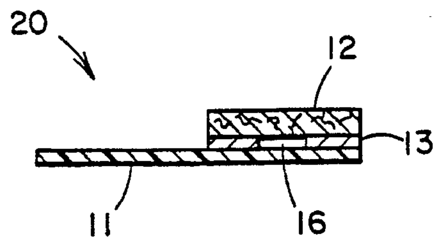


FIG. 3

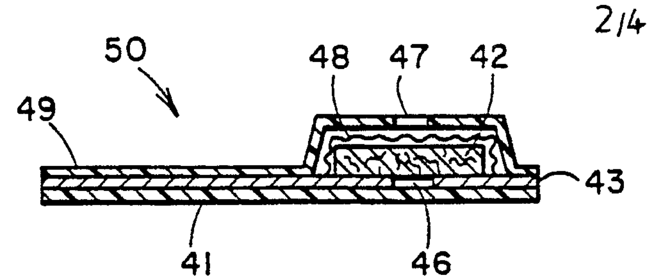


FIG. 4a

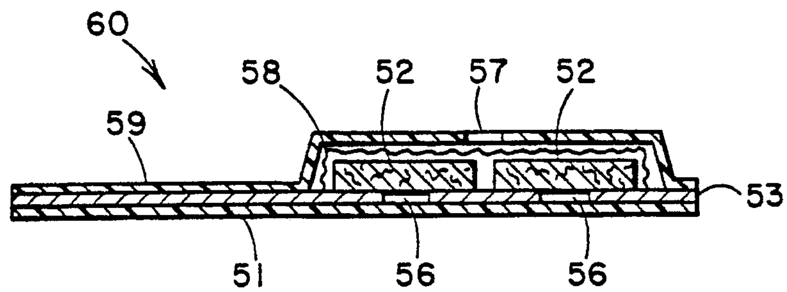


FIG. 4b

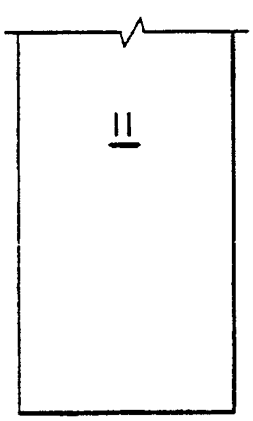


FIG. 5a

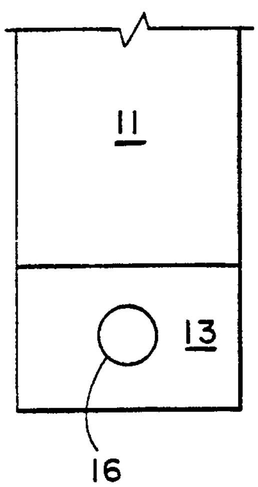


FIG. 5b

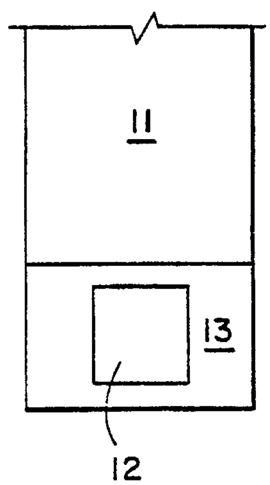


FIG. 5c

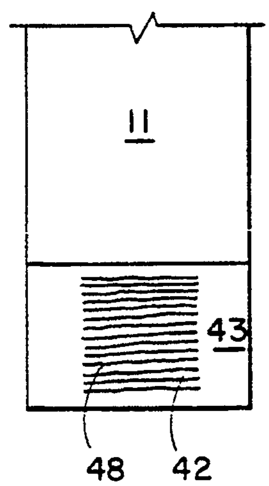


FIG. 5d

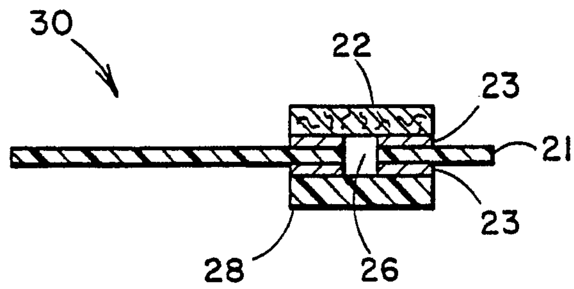


FIG. 6

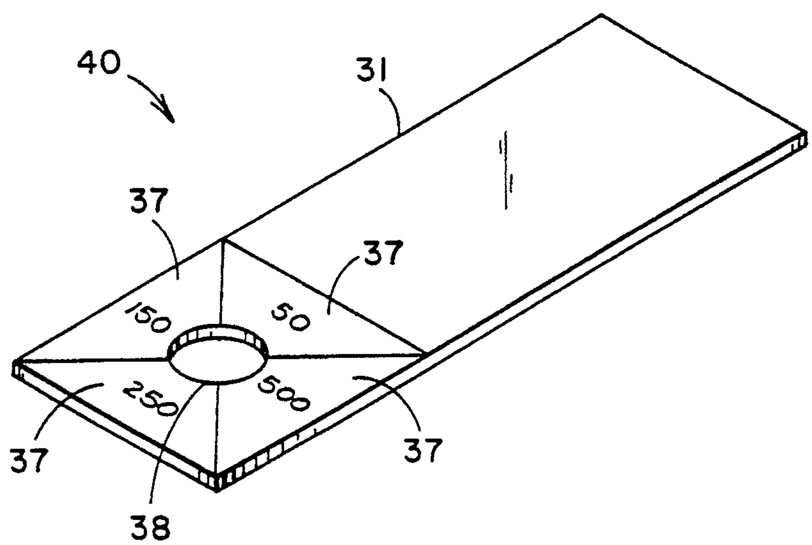


FIG. 7

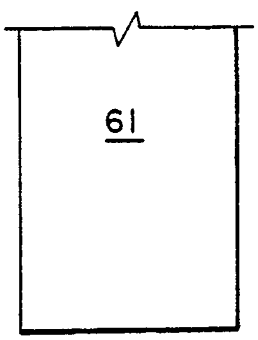


FIG. 8a

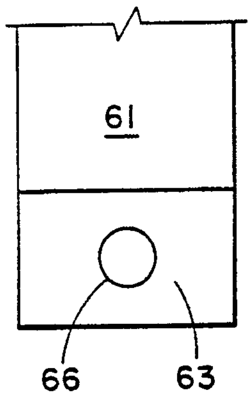


FIG. 8b

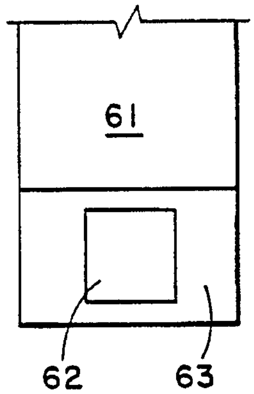


FIG. 8c

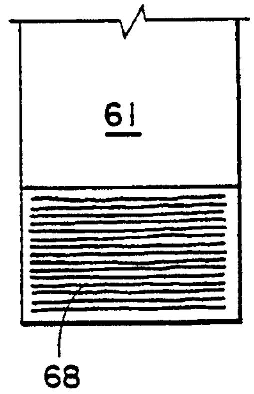


FIG. 8d

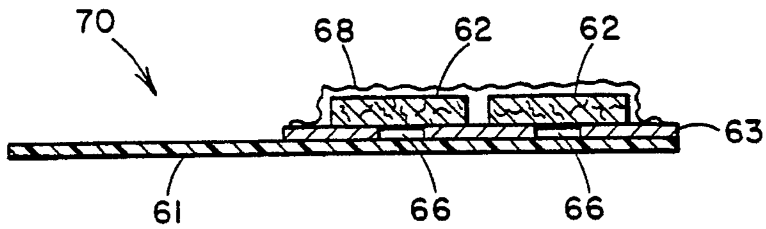


FIG. 8e

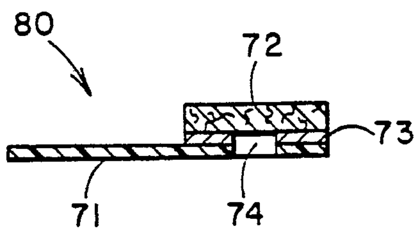


FIG. 9

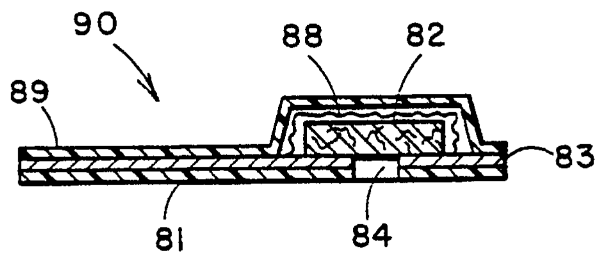


FIG. 10

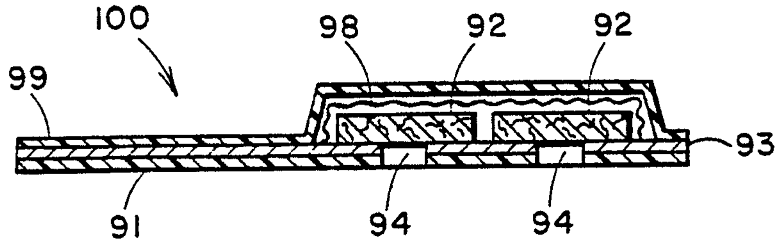


FIG. 11